(19) Europäisches Patentamt

European Patent Office

Office européen des brevets

(11) **EP 0 997 142 A1**

(12) **EUROPEAN PATENT APPLICATION**
published in accordance with Art. 158(3) EPC

(43) Date of publication:
03.05.2000 Bulletin 2000/18

(51) Int. Cl.[7]: **A61K 7/50**

(21) Application number: 99914748.1

(86) International application number:
PCT/JP99/01980

(22) Date of filing: 14.04.1999

(87) International publication number:
WO 99/53896 (28.10.1999 Gazette 1999/43)

(84) Designated Contracting States:
DE FR GB

(30) Priority: 17.04.1998 JP 10752298

(71) Applicant: Kao Corporation
Tokyo 103-8210 (JP)

(72) Inventors:
• MATSUO, Keiko,
Kao Corporation Research Labs.
Sumida-ku Tokyo 131-8501 (JP)

• UMEMOTO, Isao,
Kao Corporation Research Labs.
Sumida-ku Tokyo 131-8501 (JP)

(74) Representative: HOFFMANN - EITLE
Patent- und Rechtsanwälte
Arabellastrasse 4
81925 München (DE)

(54) **SHEET FOR CLEANING**

(57) The invention relates to a cleansing sheet obtained by impregnating a nonwoven fabric composed of a continuous long fiber with oil-absorbing powder and a lower alcohol. The sheet has high detergency to not only a stain or smear on the skin, but also sebum, can keep the skin clean, can be used irrespective of place and has good stability.

**EP 0 997 142 A1**

Printed by Xerox (UK) Business Services
2.16.7 (HRS)/3.6

**Description**

TECHNICAL FIELD

The present invention relates to a cleansing sheet which has particularly high detergency to sebum and a stain or smear on the skin, can keep the skin and hair clean, can be used irrespective of place and has good stability.

BACKGROUND ART

In order to remove a smear of sebum, it has heretofore been conducted to use a solid, pasty or liquid detergent composed mainly of a surfactant or wipe off the smear with a sheet impregnated with a liquid detergent.

However, the use of the detergent composed mainly of the surfactant has been limited to a water-supply place because the smear of sebum must be washed off with water in order to solubilize or emulsify the smear for the removal thereof, and has involved problems that a lavatory and/or clothes are stained and that the surfactant is left at positions (the margin of the hair, chin, etc.) where the detergent cannot be completely rinsed out. The sheet impregnated with the detergent has an effect of removing a stain or smear on the skin, but cannot completely remove a smear of sebum and involves problems that a sticky feel is left on the skin and that the detergency thereof is poor compared with the ordinary detergent composed mainly of the surfactant. The conventional sheet has also involved a problem that it fuzzes when the stain or smear on the skin is wiped off with the sheet, and fibers remain on the skin.

It is an object of the present invention to provide a cleansing sheet which has high detergency to not only a stain or smear on the skin, but also sebum, and low irritativeness to the skin, gives users a pleasant feeling upon use, can keep the skin clean, can be used irrespective of place and has good stability.

DISCLOSURE OF THE INVENTION

The present inventors have found that when a nonwoven fabric composed of a continuous long fiber as a fiber forming a sheet is used to impregnate it with oil-absorbing powder and a lower alcohol, a sheet which can effectively remove sebum, and at the same time has low irritativeness to the skin, gives users a pleasant feeling upon use, keeps the skin clean, can retain the skin in a cleansed state for a long period of time, can be used irrespective of place and has good stability can be obtained, thus leading to completion of the present invention.

According to the present invention, there is thus provided a cleansing sheet obtained by impregnating (a) a nonwoven fabric composed of a continuous long fiber with (b) oil-absorbing powder and (c) a lower alcohol.

According to the present inventions there is also provided the cleansing sheet further comprising an anti-fungus agent or bactericide.

BEST MODE FOR CARRYING OUT THE INVENTION

The continuous long fiber in (a) the nonwoven fabric composed of the continuous long fiber is a fiber obtained by continuous extrusion from a spinning nozzle. This continuous long fiber is preferably a cellulose fiber, with a fiber composed mainly of natural cellulose being particularly preferred. The continuous long fiber is preferably obtained by forming a sheet from a filament spun by a falling stretch spinning process. As a commercially available product of such a nonwoven fabric, there may be used a cupra nonwoven fabric, for example, "Benrize" (Asahi Chemical Industry Co., Ltd.).

The average basis weight of the nonwoven fabric is preferably 20 to 120 g/m$^2$, particularly 30 to 100 g/m$^2$, more particularly 40 to 80 g/m$^2$ in that a cleansing sheet good in touch upon use and excellent in wiping ability can be provided. The average basis weight can be determined by measuring the weight of the nonwoven fabric per fixed area (1 m$^2$) in accordance with a method known *per se* in the art.

(b) The oil-absorbing powder useful in the practice of the present invention is organic or inorganic powder and means inorganic powder or porous polymer powder capable of absorbing or occluding an oily liquid. Examples of the inorganic powder include silica, alumina, talc, kaolin, mica, mica titanium, zeolite, ultramarine blue, zinc white and iron oxide. Examples of the porous polymer powder include porous nylon powder [Orgasol 2002 (product of Elf Co.) and the like], porous vinyl polymers [for example, those described in Japanese Patent Application Laid-Open No. 316715/1988], dimethyl silicone-crosslinked elastomers [Trefil E-506C (product of Dow Corning Toray Silicone Co., Ltd.) and the like], polymethyl methacrylate [Microsphere M, Microsphere M-100, Microsphere M-300 and Microsphere M-400 (products of Matsumoto Yushi-Seiyaku Co., Ltd.) and the like], and methacrylate-alkylene dimethacrylate copolymers [Polytrap (product of Dow Corning Co.) and the like].

These oil-absorbing powders are preferably powders having an oil absorbing capacity of at least 1.0 g/g, particularly at least 2 g/g in terms of oil absorption for squalane. The oil absorption for squalane may be measured in accordance with the Measuring Method for Oil Absorption of Pigment prescribed in JIS K 5101 (1978). More specifically, a powder sample (1g) was placed on a glass sheet and kneaded by means of a spatula while squalane is being added dropwise little by little to the powder, and the time the powder has become pasty is determined to be an end point to define the amount (ml) of squalane required per 1 gram of the powder as an oil absorption. In the JIS method, boiled linseed oil is used as the oil, but squalane is used in the method according to the present invention from the viewpoint of similarity to sebum.

No particular limitation is imposed on the average particle diameter of (b) the oil-absorbing powder. However, it is preferably 0.5 to 50 μm, and spherical powder having an average particle diameter of 1 to 20 μm is preferred from the viewpoint of the touch of the resulting cleansing sheet.

The content of (b) the oil-absorbing powder in the sheet according to the present invention is preferably 0.0001 to 1 part by weight, particularly 0.0002 to 0.5 parts by weight, more particularly 0.0003 to 0.3 parts by weight, per 1 part by weight of the nonwoven fabric (a) from the viewpoints of a sebum removing effect and a feeling upon use.

Examples of (c) the lower alcohol include ethyl alcohol, isopropyl alcohol, etc., with ethyl alcohol being particularly preferred from the viewpoints of safety and odor.

The content of (c) the lower alcohol in the sheet according to the present invention is preferably 0.05 to 3 parts by weight, particularly 0.05 to 2 parts by weight, more particularly 0.1 to 2 parts by weight, per 1 part by weight of the nonwoven fabric (a) from the viewpoints of detergency to sebum and a stain or smear on the skin, and irritativeness to the skin.

The sheet according to the present invention preferably contains water from the viewpoints of irritativeness and a feel against the skin. The content of water is preferably 0.5 to 9.5 parts by weight, particularly 0.6 to 9.0 parts by weight, more particularly 0.7 to 8.5 parts by weight, per 1 part by weight of the nonwoven fabric (a).

The sheet according to the present invention may comprise an anti-fungus agent or bactericide, which is used in shampoos and the like, for the purpose of cleaning sebum and inhibiting the growth of microorganisms. Examples of the anti-fungus agent include salts (for example, calcium salt, magnesium salt, barium salt, strontium salt, zinc salt, cadmium salt, tin salt, zirconium salt, etc.) of 2-mercaptopyridine N-oxide, 1-hydroxy-2-pyridones (for example, 1-hydroxy-2-pyridone, 1-hydroxy-4-methyl-2-pyridone, 1-hydroxy-6-methyl-2-pyridone, 1-hydroxy-4,6-dimethyl-2-pyridone, 1-hydroxy-4-methyl-6-(2,4,4-trimethylpentyl)-2-pyridone, etc.), 2,2'-dithio-bis(pyridine N-oxide), trichlorocarbanilide, triclosan, sulfur, selenium sulfide, cadmium sulfide, allantoin, menthols, salicylic acid and undecylic acid. Examples of the bactericide include cationic bactericides such as benzalkonium chloride, benzethonium chloride, cetyl pyridinium chloride, chlorhexidine gluconate, chlorhexidine acetate and chlorhexidine hydrochloride, benzoic acid and salts thereof, and p-hydroxybenzoic acid esters.

These anti-fungus agents or bactericides are preferably incorporated in a proportion of 0.00001 to 0.1 parts by weight, particularly 0.00005 to 0.05 parts by weight, per 1 part by weight of the nonwoven fabric (a).

Into the sheet according to the present invention, a nonionic surfactant such as a polyoxyethylene alkyl ether, polyoxyethylene polyoxypropylene glycol, polyoxyethylene polyoxypropylene alkyl ether, partial ester of a polyhydric alcohol fatty acid, partial ester of a polyoxyethylene polyhydric alcohol fatty acid, polyoxyethylene fatty acid ester, polyglycerol fatty acid ester or polyoxyethylene hardened castor oil, a polyhydric alcohol such as glycerol or 1,3-butylene glycol, glycol ether, preservative, perfume base, coloring matter, antioxidant, pH adjuster, electrolytic substance, chelating agent and/or the like may be further suitably incorporated so far as no detrimental influence is thereby imposed on the effects of the present invention.

The sheet according to the present invention can be produced by, for example, impregnating (a) a nonwoven fabric composed of a continuous long fiber with a dispersion of oil-absorbing powder in a hydrous lower alcohol. Methods for the impregnation include a spraying method, an immersing method, etc.

In order to impregnate the nonwoven fabric (a) with the lower alcohol dispersion, it is only necessary to fold the nonwoven fabric and impregnate it with the dispersion by the spraying method. The pickup of the lower alcohol dispersion is preferably 1 to 10 g/g (nonwoven fabric), more preferably 2 to 5 g/g (nonwoven fabric) from the viewpoints of detergency and a feel upon use.

The thus-obtained cleansing sheet according to the present invention is suitable for use as a cleansing sheet for hair and skin, particularly, a face. As a method for using it, it is only necessary to wipe a face or the like with the cleansing sheet.

EXAMPLES

The present invention will hereinafter be described by the following Examples.

Example 1:

An aqueous dispersion or hydrous ethanol dispersion was impregnated [3 g/g (nonwoven fabric)] into their corresponding nonwoven fabrics shown in Table 1 by spraying so as to give their corresponding compositions shown in Table 1. The cleansing sheets thus obtained were evaluated as to detergency, a feel upon use, a stickiness-suppressing effect, wiping ability, resistance to fuzzing and the strength of the sheets in accordance with the following respective methods. The results are shown collectively in Table 1. In Table 1, the amounts of the respective components mean parts by weight per 1 part by weight of each nonwoven fabric.

(1) Measuring method of detergency:

After a certain amount of artificial sebum (model smear) was applied to the inner side of a human lower arm to measure a color difference (E1), the model smear was wiped off with each of the cleansing sheets. Thereafter, a color difference (E2) was measured again to calculate out a rate of cleansing in accordance with the following equation:

$$\text{Rate of cleansing (\%)} = (1 - E2/E1) \times 100$$

A Minolta colorimetric color difference meter CR-300 (manufactured by MINOLTA CAMERA Co., LTD.) was used as a color difference meter.

(2) Evaluating methods of the feel upon use, stickiness-suppressing effect, wiping ability, resistance to fuzzing and strength of the sheets:

Ten panelists were got to wipe off a stain or smear on their faces with each of the cleansing sheets to find an average score (criterion: ◯ where the average score was 2.5 to 3.0, △ where the average score was 1.5 to 2.4, or X where the average score was 1.0 to 1.4), thereby evaluating the sheet in accordance with the following respective evaluation standards:

(3) Evaluation standards:

① Detergency: Evaluated on the basis of the rate of cleansing (%) in accordance with the following standard:

◯:    Not lower than 80%;
△:    Not lower than 50%, but lower than 80%;
X:    Lower than 50%.

② Feel upon use: Evaluated by organoleptic evaluation as to a feeling of remaining, sticky feel and refreshed feeling after use in accordance with the following standard:

Good (preferable)    Score 3;
Fair                 Score 2;
Poor (unpreferable)  Score 1.

③ Stickiness-suppressing effect: Evaluated as to a sticky state upon elapsed time of 4 hours after wiping in accordance with the following standard:

A stickiness-free state    Score 3;
A somewhat sticky state    Score 2;
A greasy and sticky state  Score 1.

④ Wiping ability of a sheet: Evaluated as to easy removal of a stain or smear upon wiping, a feel against the skin during wiping and the like in accordance with the following standard:

Good    Score 3;
Fair    Score 2;
Poor    Score 1.

⑤ Resistance to fuzzing of a sheet: Evaluated as to fuzzing on the surface of each sheet after wiping in accordance with the following standard:

Not fuzzed          Score 3;

Somewhat fuzzed  Score 2;

Fuzzed             Score 1.

⑥ Strength of a sheet: Evaluated as to the strength of each sheet upon wiping, such as slippage, distortion or breakage, in accordance with the following standard:

Strong             Score 3;

Somewhat weak Score 2;

Weak              Score 1.

Table 1

| | | Invention product | | | Comparative product | |
|---|---|---|---|---|---|---|
| | | 1 | 2 | 3 | 1 | 2 |
| Ethanol | | 0.3 | 0.45 | 0.6 | - | 0.45 |
| Porous vinyl polymer [*1] | | 0.003 | - | - | - | 0.003 |
| Porous nylon powder [*2] | | - | 0.006 | - | - | - |
| Titanium oxide [*3] | | - | - | 0.015 | - | - |
| Silica [*4] | | - | - | - | 0.015 | - |
| Purified water | | 2.697 | 2.544 | 2.385 | 2.985 | 2.547 |
| Nonwoven fabric | Material | Cellulosic continuous fiber | | | Natural cotton fiber | |
| | Basis weight | 80 g/m$^2$ | 60 g/m$^2$ | 40 g/m$^2$ | 80 g/m$^2$ | 40 g/m$^2$ |
| Detergency | | ◯ | ◯ | ◯ | X | ◯ |
| Feel upon use | | ◯ | ◯ | ◯ | △ | △ |
| Stickiness-suppressing effect | | ◯ | ◯ | ◯ | X | ◯ |
| Wiping ability | | ◯ | ◯ | ◯ | ◯ | △ |
| Resistance to fuzzing of sheet | | ◯ | ◯ | ◯ | ◯ | X |
| Strength of sheet | | ◯ | ◯ | ◯ | ◯ | △ |

*1: Powder (average particle diameter: 3 μm; oil absorption: 3 g/g in terms of squalane) described in Japanese Patent Application Laid-Open No. 316715/1988;

*2: Orgasol 2002 (average particle diameter: 5 μm; oil absorption: 2 g/g in terms of squalane);

*3: Average particle diameter: 5 μm; oil absorption: 1 g/g in terms of squalane;

*4: Average particle diameter: 1 μm; oil absorption: 1 g/g in terms of squalane.

Example 2:

Benrize JE550 (basis weight: 55 g/m$^2$) was used as a nonwoven fabric and impregnated [3.2 g/g (nonwoven fabric)] with an impregnating solution having a composition shown in Table 2 by spraying to evaluate the resulting cleansing sheet in the same manner as in Example 1. The results are shown collectively in Table 2. In all respects of the detergency, feel upon use, stickiness-suppressing effect, wiping ability, resistance to fuzzing and strength of the sheet, the best results were demonstrated.

Table 2

| Component | | % by weight |
|---|---|---|
| Ethanol | | 14.00 |
| 1,3-Butylene glycol | | 1.00 |
| Polyoxyethylene (EO = 60) hardened castor oil | | 0.20 |
| Porous vinyl polymer [*1] | | 0.05 |
| $\ell$-Menthol | | 0.05 |
| Zinc p-phenolsulfonate | | 0.05 |
| Methylparaben | | 0.10 |
| Perfume base | | 0.02 |
| Purified water | | Balance |
| Evaluation results | Detergency | ○ |
| | Feel upon use | ○ |
| | Stickiness-suppressing effect | ○ |
| | Wiping ability | ○ |
| | Resistance to fuzzing of sheet | ○ |
| | Strength of sheet | ○ |

*1: The same polymer as that shown in Table 1.

As apparent from Tables 1 and 2, the sheets according to the present invention, which were obtained by impregnating the nonwoven fabric composed of the continuous long fiber with the oil-absorbing powder and ethanol were not only excellent in sebum-removing effect, but also good in feel upon use, and did not undergo any fuzzing.

INDUSTRIAL APPLICABILITY

The cleansing sheets according to the present invention have high detergency to not only a stain or smear on the skin, but also sebum, can keep the skin clean, can be used irrespective of place and have good stability.

**Claims**

1. A cleansing sheet obtained by impregnating (a) a nonwoven fabric composed of a continuous long fiber with (b) oil-absorbing powder and (c) a lower alcohol.

2. The cleansing sheet according to Claim 1, wherein (a) the nonwoven fabric composed of the continuous long fiber is a nonwoven fabric composed of a cellulosic continuous long fiber.

3. The cleansing sheet according to Claim 1 or 2, wherein (b) the oil-absorbing powder is composed of a porous vinyl polymer.

4. The cleansing sheet according to any one of Claims 1 to 3, wherein (c) the lower alcohol is ethyl alcohol.

5. The cleansing sheet according to any one of Claims 1 to 4, which further comprises an anti-fungus agent or bactericide.

<div style="text-align:center">

## INTERNATIONAL SEARCH REPORT

</div>

| International application No. |
| --- |
| PCT/JP99/01980 |

| A. CLASSIFICATION OF SUBJECT MATTER |
| --- |
| Int.Cl⁶ A61K7/50 |
| According to International Patent Classification (IPC) or to both national classification and IPC |

| B. FIELDS SEARCHED |
| --- |
| Minimum documentation searched (classification system followed by classification symbols)<br> Int.Cl⁶ A61K7/50 |
| Documentation searched other than minimum documentation to the extent that such documents are included in the fields searched<br> Jitsuyo Shinan Koho     1940-1992    Toroku Jitsuyo Shinan Koho    1994-1996<br> Kokai Jitsuyo Shinan Koho    1970-1992 |
| Electronic data base consulted during the international search (name of data base and, where practicable, search terms used) |

| C. DOCUMENTS CONSIDERED TO BE RELEVANT | | |
| --- | --- | --- |
| Category* | Citation of document, with indication, where appropriate, of the relevant passages | Relevant to claim No. |
| Y | Microfilm of the specification and drawings annexed to the request of Japanese Utility Model Application No. 62-49324 (Laid-open No. 63-155494) (Kenji Nakamura), 12 October, 1988 (12. 10. 88) (Family: none) | 1-4 |
| Y | JP, 2-36114, A (Kenji Nakamura), 6 February, 1990 (06. 02. 90) (Family: none) | 1-4 |
| Y | JP, 6-319664, A (Tokai Pulp K.K.), 22 November, 1994 (22. 11. 94) (Family: none) | 1-4 |
| Y | Microfilm of the specification and drawings annexed to the request of Japanese Utility Model Application No. 1-21270 (Laid-open No. 2-115530) (Shiraimatsu Shinyaku K.K.), 17 September, 1990 (17. 09. 90), Particularly page 5, lines 18, 19 ; pages 14 to 18 ; Figs. 1, 2 (Family: none) | 1-4 |

☐ Further documents are listed in the continuation of Box C.     ☐ See patent family annex.

| | |
| --- | --- |
| *   Special categories of cited documents:<br>"A"   document defining the general state of the art which is not considered to be of particular relevance<br>"E"   earlier document but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| 8 June, 1999 (08. 06. 99) | 22 June, 1999 (22. 06. 99) |
| Name and mailing address of the ISA/<br> Japanese Patent Office | Authorized officer |
| Facsimile No. | Telephone No. |

Form PCT/ISA/210 (second sheet) (July 1992)